# EUROPEAN PATENT APPLICATION

(11) **EP 4 389 051 A1**
(43) Date of publication of application: **26.06.2024**
(21) Application number: 22307009.5
(22) Date of filing: 22.12.2022
(51) Int. Cl.: A61B 34/30, A61B 17/3207, A61B 17/00

(54) **NOSE DIVE MICROROBOT**

(71) Applicant: Robeaute, 75002 Paris (FR)
(72) Inventor: CHALIVOY, Lucas, 75002 Paris (FR); DUPLAT, Bertrand, 75002 Paris (FR); FRANCOIS, Quentin, 75002 Paris (FR); PLANARD, Philippe, 75002 Paris (FR); OULMAS, Ali, 75002 Paris (FR)
(74) Representative: Icosa

(57) **Abstract**

The present invention relates to a microrobot (10) configured to move in a viscoelastic anatomic environment along a propulsion direction, comprising
- a body (12) extending along a body axis (X),
- a navigation head (14) extending along a head axis (H) presenting a given configuration with regards to the body,
- a propulsion element (16) extending along a propulsion axis (A), and presenting an external surface (18) with a helical external thread (20) presenting a given configuration with regards to the body,
- driving means (22) configured to drive the propulsion element in rotation around the propulsion axis (A),

The microrobot comprises an orientation arrangement (24) configured to change at least one of the given configurations of the navigation head and of the external thread with regards to the body in order to change the propulsion direction.

## Description

### FIELD OF INVENTION

The present invention relates to a microrobot configured to navigate inside a solid viscoelastic material by means of crack generation and propagation inside said viscoelastic material.

### BACKGROUND OF INVENTION

The ability to reach deep and functional structures without damage is a major challenge in mini-invasive surgery, especially in neurosurgery.

Thanks to micro-technologies, it becomes possible to send a fully autonomous microrobot inside an organ of a subject, such as a brain. However, most existing microrobots in the state of the art are only able to propel, by means of a propeller, in viscous liquids as for example blood or cerebrospinal fluid. The brain, on the other hand, is well known to be, at least partially, a solid viscoelastic organ.

In materials science and continuum mechanics, viscoelasticity is the property of materials that exhibit both viscous and elastic characteristics when undergoing deformation. Viscous materials, like water, resist shear flow and strain linearly with time when a stress is applied. Elastic materials strain when stretched and immediately return to their original state once the stress is removed. Viscoelastic materials have elements of both of these properties and, as such, exhibit time-dependent strain. Sometimes, a viscoelastic material can be assimilated to a solid material.

Thus, the propulsion of a micro robot by means of a propeller in a solid viscoelastic environment, as is the brain, is very difficult because the brain is elastic (it can store energy and restore it to the environment). Using such a propeller in the brain should meet specific criteria to be able to surpass this elastic barrier (which is a characteristic of the material) and move into it, otherwise the propeller and the micro robot will remain at the same place, blocked into the material.

Effectively penetrating and moving through viscoelastic biological tissues is thus a challenge.

In this context, the invention is intended to propose a microrobot having an efficient propulsion mechanism in a solid viscoelastic environment like the brain. To be able to move inside a solid viscoelastic material, a microrobot has to firstly open (or generate) a crack, then has to widen it to be able to enter it, and eventually enter it to be able to move inside said environment.

Another important requirement is that the microrobot should be capable of moving in an organ while limiting as much as possible the physiological damage that its passage causes to the organ. It is thus important that the generated and propagated crack is as precisely oriented as possible and as minimally large as possible.

Another important requirement is that the microrobot should be capable of moving in an organ while limiting as much as possible the physiological damage that its passage causes to the organ.

In order to respond to those technical and biological constraints, for medical and surgical purposes, in the solid matter of the brain, the extracellular matrix of the brain parenchyma, but also in other viscoelastic organs such as the pancreas or the liver, an energy-efficient means of propulsion is the propagation of a crack by skillful insertion of a device and in particular, the insertion of a microrobot inside here-above listed organs or organ regions.

Such a microrobot should be technically equipped and configured to at least propagate an existing crack and, in some embodiments, to even generate a crack in which it could then sneak in in order to navigate inside the organ or organ region.

To propagate such a crack, it is possible to use a rotating end (but not exclusively) comprising a screw pitch. The rotating screwed pitch can thus, by rotating inside the material, spread an existing crack (or create a new one) and extends its opening further in front, while allowing the microrobot to advance like a screw.
The aim of this invention is to propose a microrobot technically able to navigate biological viscoelastic environments in a controlled way along a navigation route as little invasive and damaging as possible.

### SUMMARY

This invention thus relates to a microrobot configured to move in a viscoelastic anatomic environment along a propulsion direction, the microrobot comprising
- a body extending along a body axis,
- a navigation head extending along a head axis between a free distal tip and a proximal base the navigation head presenting a given configuration with regards to the body,
- a propulsion element extending along a propulsion axis, and presenting an external surface with a helical external thread, the external thread presenting a given configuration with regards to the body,
- driving means configured to drive the propulsion element in rotation around the propulsion axis with respect to the body,
The microrobot further comprises an orientation arrangement configured to change at least one of the given configurations of the navigation head and of the external thread with regards to the body in order to change the propulsion direction of the micro-robot.

This way, the solution enables to reach the here-above mentioned objective. Especially, it enables to change the propulsion direction of the micro-robot in a controlled way and thus to precisely control the navigation route of the microrobot in a viscoelastic environment.

The system according to the invention may comprises one or several of the following features, taken separately from each other or combined with each other:
- the driving means may further be configured to also drive the navigation head in rotation,
- the given configuration of the external thread may include a given tilting angle of the head axis with respect to the body axis, and the orientation arrangement may be configured to change the given tilting angle of the head axis with respect to the body axis,
- the orientation arrangement may comprise a pivotal connection configured to rotate the navigation head with regards to the body along at least one pivot axis,
- the given configuration of the external thread of the propulsion element may include a given tilting angle of the propulsion axis with respect to the body axis, and the orientation arrangement may be configured to change the given tilting angle of the propulsion axis with respect to the body axis,
- the orientation arrangement may comprise a pivotal connection configured to rotate the propulsion element with regards to the body along at least one pivot axis,
- the driving means may comprise a support shaft connecting the proximal base of the navigation head to the body and comprising the pivotal connection,
- the support shaft may be secured to the engine body by means of a piece of flexible material,
- the support shaft may be made of flexible material,
- the support shaft may also connect the propulsion element to the body,
- the configuration of the external thread may include a pitch of the external thread, and the orientation arrangement may be configured to change said pitch of the external thread,
- the orientation arrangement may comprise a series of connectors movably mounted inside the propulsion element and connected to the external thread, the connectors having a relative positioning with respect to each other, the relative positioning varying according to an angular position with regards to external surface of the propulsion element,
- the series of connectors may be configured so that the relative position of the connectors with regards to the external surface of the propulsion element varies between:
   ∘ a first relative position in which the external surface is in a first relative angular position, the connectors in the first relative position being spaced apart of respective first distances from each other, the first distances being minimum,
   ∘ a second relative position in which the external surface is in a second relative angular position, the connectors in the second relative position being spaced apart of respective second distances from each other, the second distance being maximum,
- the series of connectors may comprise:
   ∘ an internal central connector localized inside the propulsion element, the central connector being tiltable, inside the propulsion element, with regards to the propulsion axis
   ∘ at least two transversal connectors extending between the central connector and the external thread.

### BRIEF DESCRIPTION OF THE DRAWINGS

The invention will be better understood, and other aims, details, characteristics and advantages thereof will emerge more clearly on reading the detailed explanatory description which follows, of embodiments of the invention given by way of illustration, purely illustrative and non-limiting examples, with reference to the accompanying drawings:
- **figure 1a** is a schematical view of the present invention according to a first embodiment,
- **figure 1b** is the same schematical view than 1a presenting a different given configuration,
- **figure 2** is a detailed schematic view of a support shaft according to one embodiment of the present invention,
- **figures 3a** and **3b** are schematical views presenting two different given configurations of a second embodiment of the present invention,
- **figures 4a and 4b****,** are schematical views presenting two different given configurations of a third embodiment of the present invention,
- **figure 5** is a detailed schematic view of a particular orientation arrangement according to one embodiment of the present invention,
- **figures 6a and 6b****,** are schematical views presenting two different given configurations of a fourth embodiment of the present invention,
- **figures 7a and 7b****,** are schematical views presenting two different given configurations of a fifth embodiment of the present invention.

### DETAILED DESCRIPTION

The present invention, as can be seen on the different figures is about a microrobot 10 configured to move in a viscoelastic anatomic environment along a propulsion direction.

This viscoelastic environment can for example be the extracellular matrix of the brain.

According to the present invention, the microrobot 10 comprises:
- a body 12 extending along a body axis X,
- a navigation head 14 extending along a head axis H between a free distal tip 14a and a proximal base 14b,
- a propulsion element 16 extending along a propulsion axis A, and presenting an external surface 18 with a helical external thread 20,
- driving means 22 configured to drive the propulsion element 16 in rotation around the propulsion axis A with respect to the body 12.

The driving means 22 comprise the rotor (or moving parts) of an activable motor (not represented) situated inside the body 12. The activable motor comprises a stator and a rotor. The stator is part of the body 12.

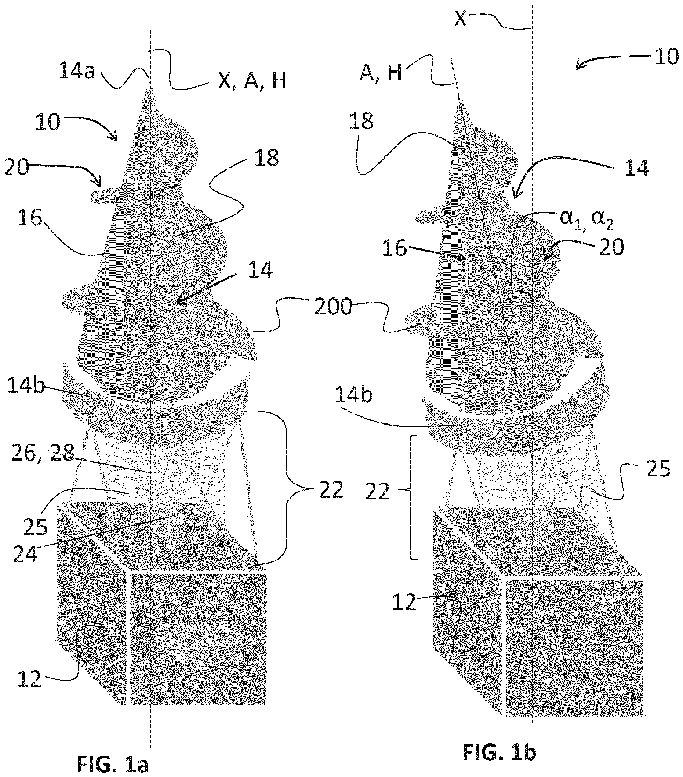

The driving means 22 further comprise movement transmission means. Those movement transmission means are connected to the activable motor and enable the movement generated by the activated motor to be transmitted to elements of the microrobot 10 which need to be put in motion. More particularly, the driving means 22 are configured to drive the propulsion element 16 in rotation. In the embodiments of figures 1a, 1b, 3a, 3b, 4a, 4b 6a and 6b, the driving means 22 are further configured to also drive the navigation head 14 in rotation. In those embodiments, the navigation head 14 and the propulsion element 16 are the same technical element.

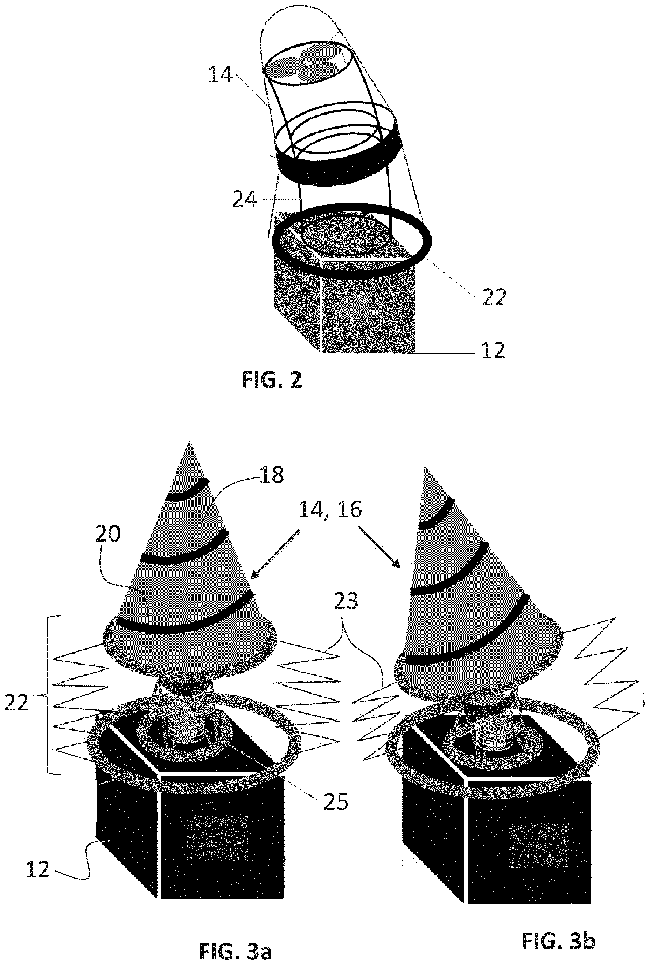

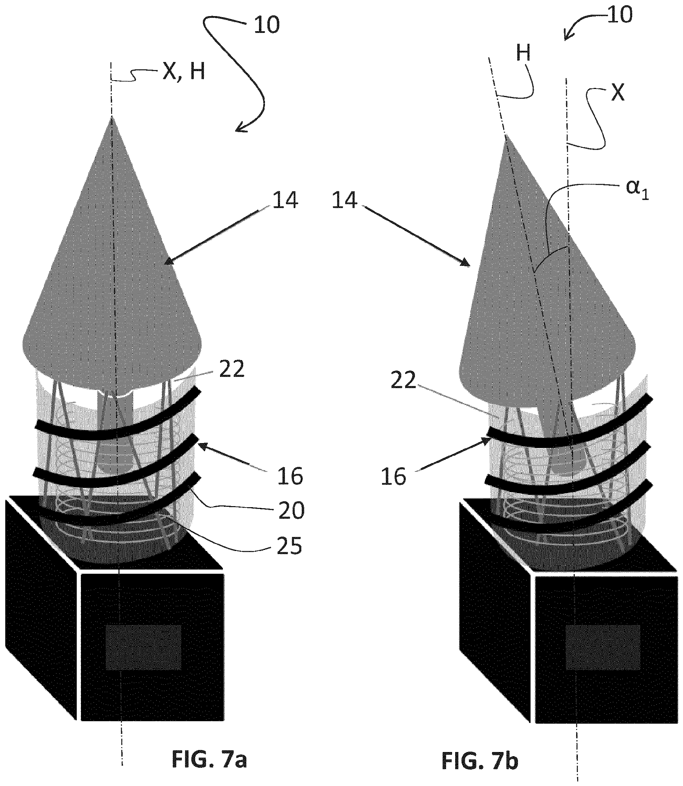

The movement transmission means of the driving means 22 can for example be a series of threads made of smart material, for example nitinol or an electro-active polymer like PEDOT, regularly distributed around the body axis X (see figures 1a, 1b and 7a, 7b). In the embodiment of figures 3 a and 3b, those threads are further coupled to a central string structure optimizing the performance of the orientation. In a further embodiment, not shown, the driving means 22 may comprise a central driving rod. In such an embodiment, the support shaft 24 might be part of the driving means 22. The driving means 22 can further comprise an external driving cylinder directly connected to the motor and further connecting the proximal base 14b of the navigation head 14. This external driving cylinder can be connected to the proximal base 14b of the navigation head by means of a bellow shaped element 23 (see for example figures 3a and 3b). This bellow shaped element 23 may also be a string. A bellow present nevertheless the technical advantage to be a closed element and can thus act as a sort of shell or envelope, thus enabling to protect the elements situated within the bellow from the viscoelastic environment. When the rotation of the propulsion element 16 comes from the periphery of the motor of the body 12, it presents the technical advantage of transmitting a stronger torque to the rotating propulsion element 16.

In some embodiments the navigation head 14 is connected to the rotor of the activable motor by means of a support shaft 24 connecting the proximal base 14b of the navigation head 14 to the body 12. The support shaft 24 extends along the body axis X. In some embodiments, the propulsion element 16 is also connected to the rotor of the activable motor by means of the support shaft 24. In those embodiments, the navigation head 14 and the propulsion element 16 are preferably (but not necessarily) the same technical element. In some alternative embodiments, for example the embodiment of figures 7a and 7b, the propulsion element 16 extends around the support shaft 24.

In some alternative embodiments, the navigation head 14 is connected to the rotor of the activable motor by means of a bellow or string shaped element 25. In a resting position, the bellow or string shaped element 25 extends along the body axis X. Because of its structure, the bellow or string shaped element 25 allows the navigation head 14 to be easily and smoothly tilted in any possible direction with regards to the body 12. In some embodiments, the propulsion element 16 is also connected to the rotor of the activable motor by means of the bellow or string shaped element 25. In some embodiments, the bellow or string shaped element 25 surrounds the support shaft 24.

The body 12 is connected to the stator of the activable motor. This stator doesn't move in orientation. More precisely, the stator doesn't turn on itself along the body axis X. Its technical purpose is to serve as the orientation reference. The body 12 can carry multiple functional elements such as electronics, sensors, drug cargo, or other tools or elements.

The navigation head 14 presents a general conical or spiky shape. The free distal tip 14a of the navigation head 14 presents thus a diameter which is significantly smaller than the diameter of the proximal base 14b of the navigation head 14. This shape can be slightly twisted or bent. More precisely, the free distal tip 14a can present a variable length and can be bent and/or twisted along its length. The shape can be conical, arrow-like, convex or concave.

The function of the navigation head 14 is to propagate and sometimes, depending on the embodiment, create a crack in the viscoelastic environment in which the microrobot 10 has to navigate. The shape of the navigation head 14 influences the general shape of the generate crack and must therefore present some specific technical features. The main technical features of the navigation head 14 are its sharpness and its friction coefficient with the viscoelastic medium (which should be minimum). As the microrobot 10 cannot navigate inside a solid material (elastic part of a viscoelastic environment), it is necessary to generate a crack inside said environment. The microrobot 10 can then navigate inside said crack towards its destination.

Unlike the usual crack theory, where the crack propagation is done in a straight line, the microrobot 10 according to the present invention has to be able to rotate/steer in all possible directions, right/left and up/down, to change its trajectory. The microrobot thus, tilts its navigation head 14 towards a specific direction to orientate the crack. The microrobot 10 then moves forward in this specific direction to propagate the crack in this specific direction.

The microrobot 10 according to the present invention thus enables to control its trajectory inside the viscoelastic material by:
- controlling the crack orientation by means of controlling the tilting of the navigation head 14 before generating the crack,
- controlling the propulsion of the microrobot 10 inside the generated crack, thus controlling the crack propagation.

Regardless of its shape, the navigation head 14 presents, at all time, a given configuration with regards to the body 12. The concept of configuration in the context of the present application will be further detailed below.

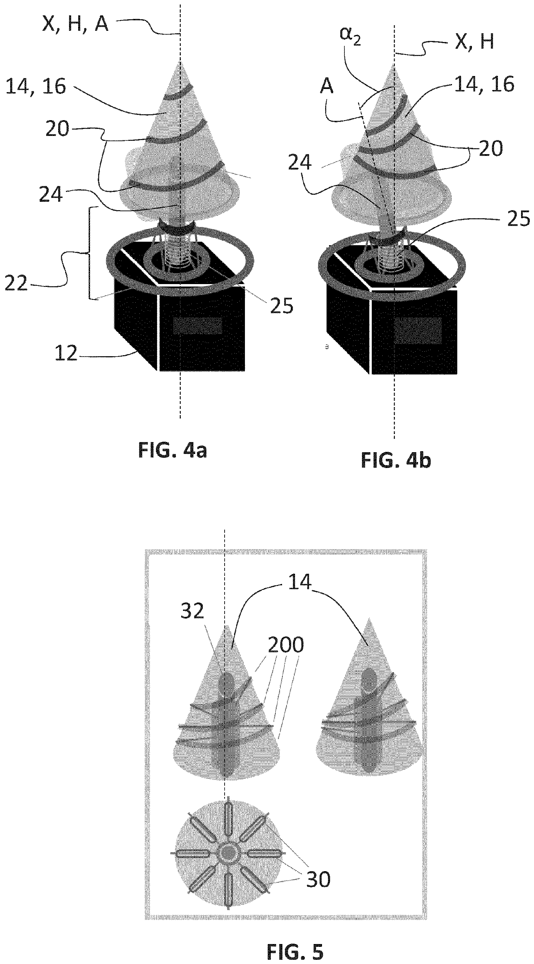

In some embodiments, the navigation head 14 and the propulsion element 16 are the same technical element. More specifically, in those embodiments, the navigation head 14 forms a free distal end, or front tip, of the propulsion element 16 (see figures 1a, 1b or 4a, 4b, for example), and the propulsion element 16 presents a general conical shape.

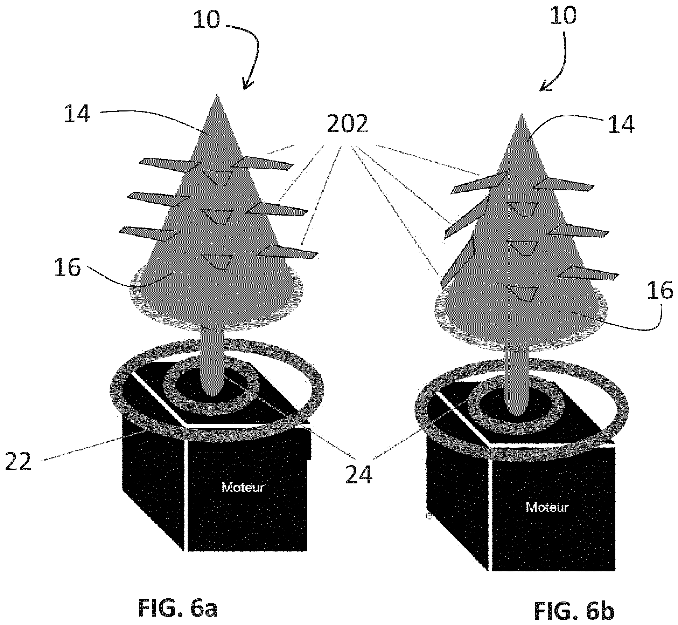

As already stated above, the propulsion element 16 presents an external surface 18 with a helical external thread 20 (see figures 1a, 1b, 2a, 2b or 7a, 7b, for example). Depending on the embodiment, the external thread 20 can be formed by a single continuous helicoidal blade 200 (see figures 1a and 1b) or by a series of small blades 202 all aligned in a helicoidal way around the external surface 18 of the propulsion element 16 (see figures 6a and 6b). Similarly to the navigation head 14, the external thread 20 presents, at all time, a given configuration with regards to the body 12.

Once the given configuration of the external thread 20 is defined and once the propulsion element 16 is put into motion (a rotational motion) by the driving means 22, the propulsion element 16 works as a helix or an impeller and enables the microrobot 10 to move along the propulsion direction. The microrobot 10 according to the present invention can thus be compared to a system that works like a screw penetrating a solid material like a wall or a wooden board.

In the present invention, the wording "configuration" designates, as defined by the Collins Online Dictionary: "an arrangement of a group of things". The wording "configuration" thus includes relative positions of elements with regards to each other. Depending on the embodiments, the configuration of the navigation head 14 with regards to the body 12, and the configuration of the external thread 20 of the propulsion element 16 with regards to the body 12 can be either independent from each other (meaning that the configuration of the navigation head 14 can be changed without affecting the configuration of the external thread 20 of the propulsion element 16 and vice versa) or interdependent to each other (meaning that a modification of the configuration of the navigation head 14 induces a modification of the configuration of the external thread 20 and vice versa).

Considering the embodiments of 7a and 7b, the given configuration of the navigation head 14 includes a given tilting angle α₁ of the head axis H with regards to the body axis X.

Considering the embodiments of figures 1a, 1b, and 2a, 2b, the given configuration of the external thread 20 of the propulsion element 16 with regards to the body 12 includes a given tilting angle α₂ of the propulsion axis A of the propulsion element 16 with respect to the body axis X.

In the embodiments of figures 1a, 1b,2a and 2b, the navigation head 14 is part of the propulsion element 16 and the given titling angles α₁ and α₂ are the same angle. The given configuration of the navigation head 14 and of the external thread 20 are interdependent and one cannot be changed without changing the other.

In the embodiment of figures 7a and 7b the propulsion element 16 does never change its configuration with regards of the body 12. The given configurations of the external thread 20 and of the navigation head 14 are thus independent from each other.

Considering the embodiment of 4a, 4b, 6a and 6b, the configuration of the external thread 20 of the propulsion element 16 with regards to the body 12 includes a pitch of the external thread 20. The pitch of the external thread 20 is thus variable along the external surface 18 of the propulsion element 16.

The change of the given configuration either of one or both of the navigation head 14 and/or the external thread 20 enables to change the propulsion direction of microrobot 10 while it moves forward inside the viscoelastic environment. This configuration change either enables to generate a new crack presenting a desired orientation inside the viscoelastic environment or enables the microrobot 10 to smoothly follow the way designed by an already existing crack (precedingly generated by the microrobot 10 or not).

In order to change the given configuration of the navigation head 14 and/or of the external thread 20 of the propulsion element 16 with regards to the body 12, the microrobot 10 comprises an orientation arrangement 26. The orientation arrangement 26 comprises a series of elements interacting together in order to change the given configurations of either one or both of the external thread 20 and the navigation head 14. The orientation arrangement 26 is thus, broadly speaking, configured to change the propulsion direction of the moving microrobot 10. Reworded the other way around, the change of propulsion direction is thus achieved by changing the given configuration of at least one between the external thread 20 or the navigation head 14.

In some embodiments, the orientation arrangement 26 is more precisely configured to change the given tilting angle α₂ of the propulsion axis A of the propulsion element 16 with respect to the body axis X. The orientation arrangement 26 may also be configured to change the given tilting angle α₁ of the head axis H of the navigation head 14 with respect to the body axis X.

In those embodiments, the orientation arrangement 26 may comprise a pivotal connection 28 configured to tilt the navigation head 14 with regards to the body 12 along at least one pivot axis. In some other of those embodiments, the orientation arrangement 26 may comprise a pivotal connection 28 configured to tilt the propulsion element 16 with regards to the body 12 along the at least one pivot axis. In those embodiments, the navigation head 14 is preferably part of the propulsion element 16. In some of those embodiments, it is the support shaft 24 which comprises the pivotal connection 26 enabling the navigation head 14 to be tilted with regards to the body 12 (see figures 1a and 1b). In some embodiments, the bellow or string shaped element 25 is part of the pivotal connection 26. In some alternative embodiment, the support shaft 24 is secured to the rotor of the activable motor by means of a piece of flexible material (see figure 7a, 7b), for example a 3D printed resin, a given polymer, or PEEK.

In some further alternative embodiment, the support shaft 24 is made of flexible material (see figure 2). In some embodiment, the support shaft 24 comprises smart material threads as described further above, and those smart material threads thus allow to tilt the navigation head 14 (and sometimes the propulsion element 16) when activated in a specific direction. In some alternative embodiment illustrated on figure 2, the support shaft 24 comprises a series of internal conducts. Each conduct is put under pressure and in varying the pressure of one or several of those internal conducts, some tilting of the support shaft 24 can be achieved.

Generally speaking, the orientation arrangement 26 is part of a compliant mechanism. In mechanical engineering, a compliant mechanism is defined as a flexible mechanism that achieves force and motion transmission through elastic body deformation. It gains some or all of its motion from the relative flexibility of its members rather than from rigid-body joints alone. The benefit of such a compliant mechanism is that there aren't two (or more) parts which move relative to each other. This generally increases the robustness of the system but limits its ability to move

Considering the embodiments of figures 4a, 4b and 6a, 6b, the orientation arrangement 26 is configured to change the pitch of the external thread 20 of the propulsion element 16. More precisely, regarding those embodiments, the orientation arrangement 26 comprises a series of connectors 30, 32 movably mounted inside the propulsion element 16 and connected to the external thread 20. More precisely, the orientation arrangement 26 comprises:
- a central internal connector 32 localized inside the propulsion element 16, the central connector 32 being tiltable, inside the propulsion element 16, with regards to the propulsion axis A,
- at least two transversal connectors 30 extending between the central connector 32 and the external thread 20.

All of the connectors 30 thus present a relative positioning with respect to each other, the relative positioning varying according to an angular position with regards to the external surface 18 of the propulsion element 16. In the embodiments in which the propulsion element 16 presents a single helicoidal blade 200, the connectors 30 work a in combination to change the pitch of said single blade 200. In the embodiments in which the propulsion element 16 presents a series of small blades 202, each small blade 202 is connected to at least one connector 30 and its pitch can be changed independently from the other small blades 202. This second mechanism resembles the mechanism of helicopter blade pitch modification.

Regardless of the shape of the external thread 20, the series of connectors 30 is configured so that the relative positioning of the connectors 30 varies between:
- a first relative position in which the external surface 18 is in a first relative angular position,
- a second relative position in which the external surface 18 is in a second relative angular position.
In the first relative position, the connectors 30 are spaced apart from each other of a first distance from each other, the first distances being minimum. In the second relative position, the connectors 30 are spaced apart of a second distances from each other, the second distance being maximum.

This specific embodiment, enables to achieve an asymmetrical change of the pitch of the external thread 20. The orientation of the propulsion element 16 and of the navigation head 14 are not modified, but the given configuration of the external thread 20 is nevertheless modified and the microrobot 10 presents, on one side of the external surface 18 of the propulsion element 16 a pitch which is tighter while, simultaneously, on the other opposite side of the external surface 18, the pitch is further away.

## Claims

1. Microrobot (10) configured to move in a viscoelastic anatomic environment along a propulsion direction, the microrobot (10) comprising
- a body (12) extending along a body axis (X),
- a navigation head (14) extending along a head axis (H) between a free distal tip (14a) and a proximal base (14b) the navigation head (14) presenting a given configuration with regards to the body (12),
- a propulsion element (16) extending along a propulsion axis (A), and presenting an external surface (18) with a helical external thread (20), the external thread (20) presenting a given configuration with regards to the body (12),
- driving means (22) configured to drive the propulsion element (16) in rotation around the propulsion axis (A) with respect to the body (12),
**wherein** the microrobot (10) further comprises an orientation arrangement (24) configured to change at least one of the given configurations of the navigation head (14) and of the external thread (20) with regards to the body (12) in order to change the propulsion direction of the micro-robot (10).

2. Microrobot (10) according to the preceding claim, **wherein** the driving means (22) are further configured to also drive the navigation head (14) in rotation.

3. Microrobot (10) according to any one of the preceding claims, **wherein** the given configuration of the external thread (20) includes a given tilting angle (α₁) of the head axis (H) with respect to the body axis (X), and **wherein** the orientation arrangement (26) is configured to change the given tilting angle (α₁) of the head axis (H) with respect to the body axis (X).

4. Microrobot (10) according to the preceding claim, **wherein** the orientation arrangement (26) comprises a pivotal connection (28) configured to rotate the navigation head (14) with regards to the body (12) along at least one pivot axis.

5. Microrobot (10) according to any one of the preceding claims, **wherein** the given configuration of the external thread (20) of the propulsion element (16) includes a given tilting angle (α₂) of the propulsion axis (A) with respect to the body axis (X), and **wherein** the orientation arrangement (26) is configured to change the given tilting angle (α₂) of the propulsion axis (A) with respect to the body axis (X).

6. Microrobot (10) according to the preceding claim, **wherein** the orientation arrangement (26) comprises a pivotal connection (28) configured to rotate the propulsion element (16) with regards to the body (12) along at least one pivot axis.

7. Microrobot (10) according to anyone of the preceding claims, **wherein** the driving means (22) comprise a support shaft (24) connecting the proximal base (14b) of the navigation head (14) to the body (12) and comprising the pivotal connection (28).

8. Microrobot (10) according to the preceding claim, **wherein** the support shaft (24) is secured to the engine body (12) by means of a piece of flexible material.

9. Micro-engine according to claims **7** or **8, wherein** the support shaft (24) is made of flexible material.

10. Microrobot (10) according to claims **7** to **9, wherein** the support shaft (24) also connects the propulsion element (16) to the body (12).

11. Microrobot (10) according to any of the preceding claims, **wherein** the configuration of the external thread (20) includes a pitch of the external thread (20), and **wherein** the orientation arrangement (26) is configured to change said pitch of the external thread (20).

12. Microrobot (10) according to the preceding claim, **wherein** the orientation arrangement (26) comprises a series of connectors (30) movably mounted inside the propulsion element (16) and connected to the external thread (20), the connectors (30) having a relative positioning with respect to each other, the relative positioning varying according to an angular position with regards to external surface (18) of the propulsion element (16).

13. Microrobot (10) according to the preceding claim, **wherein** the series of connectors (30) are configured so that the relative position of the connectors (30) with regards to the external surface (18) of the propulsion element (16) varies between:
- a first relative position in which the external surface (18) is in a first relative angular position, the connectors (30) in the first relative position being spaced apart of respective first distances from each other, the first distances being minimum,
- a second relative position in which the external surface (18) is in a second relative angular position, the connectors (30) in the second relative position being spaced apart of respective second distances from each other, the second distance being maximum.

14. Microrobot (10) according to claims **12** or **13, wherein** the series of connectors (30) comprises:
- an internal central connector (32) localized inside the propulsion element (16), the central connector (32) being tiltable, inside the propulsion element (16), with regards to the propulsion axis (A),
- at least two transversal connectors (30) extending between the central connector (32) and the external thread (20).
